# EUROPÄISCHE PATENTSCHRIFT

(11) **EP 2 803 384 B1**
(45) Veröffentlichungstag und Bekanntmachung des Hinweises auf die Patenterteilung: **31.08.2016**
(21) Anmeldenummer: 14162276.1
(22) Anmeldetag: 28.03.2014
(51) Int. Cl.: A61N 1/362, A61N 1/368

(54) **Externer Herzschrittmacher zur biatrialen Stimulierung**
External pacemaker for biatrial stimulation
Stimulateur cardiaque externe pour stimulation biauriculaire

(30) Priorität: 15.05.2013 DE 102013008317; 11.09.2013 DE 102013015074
(43) Veröffentlichungstag der Anmeldung: 19.11.2014
(73) Patentinhaber: Osypka AG, 79618 Rheinfelden-Herten (DE)
(72) Erfinder: Tröger, Tobias, 91052 Erlangen (DE); Braun, Michael, 89231 Neu-Ulm (DE); Göttsche, Thorsten, 79618 Rheinfelden-Herten (DE); Loesch, Thomas, 41179 Mönchengladbach (DE)
(74) Vertreter: Herrmann, Uwe

(56) Entgegenhaltungen:
- EP-A1- 1 350 539
- US-A1- 2003 195 579
- US-A1- 2006 287 685
- US-A1- 2011 125 049

## Beschreibung

Die Erfindung betrifft einen externen Herzschrittmacher zur kardialen Stimulation, wobei der Herzschrittmacher eine Steuer- oder Regeleinheit zur Abgabe wenigstens eines Stimulationspulses aufweist.

Im Rahmen von herzchirurgischen Eingriffen werden häufig externe Herzschrittmacher eingesetzt, die zur temporären kardialen Stimulation und intrakardialen EKG-Überwachung dienen. Zur Stimulation und Überwachung werden Elektroden eingesetzt, die auf dem Herzmuskel aufgenäht sind und über Herzdrähte mit dem externen Herzschrittmacher verbunden sind.

Der Output des Schrittmachers wird bestimmt durch Impulsamplitude (in Volt) und Impulsdauer. Die Impulsstärke wird durch die Impulsamplitude wiedergegeben und die Impulsbreite gibt die Dauer des Impulses an. Sie wird üblicherweise in Millisekunden angegeben.

Um dem Patienten nicht unnötig Energie zuzuführen, wird vor Aufnahme des Stimulationsbetriebs die Reizschwelle für das Herz bestimmt. Die Reizschwelle definiert den minimalen Amplitudenwert des Stimulationspulses, bei dem eine ausreichende Stimulationswirkung des Herzens erreicht bzw. erkannt wird. Die Reizschwelle kann dabei für die unterschiedlichen Herzbereiche, d.h. Ventrikel bzw. Atrium, unterschiedlich ausfallen. Auch können Abweichungen zwischen dem linken und rechten Ventrikel bzw. Atrium auftreten.

Zukünftig werden verstärkt biatriale Schrittmachermodi zum Einsatz kommen, bei denen der rechte und/oder der linke Vorhof überwacht und, sofern keine Herzeigenaktivität erkannt wird, ein Stimulationspuls abgegeben wird. Diese Vorgehensweise dient insbesondere dazu, die Wahrscheinlichkeit eines Vorhofflimmerns zu reduzieren. Bisher sind keine externen Herzschrittmacher für die biatriale Stimulation bekannt. Zudem wird regelmäßig eine gemeinsame Reizschwelle und eine gemeinsame Stimulationsamplitude für das linke und rechte Atrium bestimmt und abgegeben.

Die Aufgabe der vorliegenden Erfindung besteht darin, einen externen Herzschrittmacher aufzuzeigen, der für die biatriale Stimulation geeignet ist und zudem die Effektivität der biatrialen Stimulation optimiert.

Diese Aufgabe wird durch einen externen Herzschrittmacher mit den Merkmalen des Anspruchs 1 gelöst. Vorteilhafte Ausgestaltungen des Herzschrittmachers sind Gegenstand der abhängigen Ansprüche 2 bis 9.

Gemäß Anspruch 1 wird also ein externer Herzschrittmacher zur biatrialen Stimulierung vorgeschlagen, der eine Steuer- oder Regeleinheit zur Abgabe wenigstens eines Stimulationspulses aufweist. Die Stimulationspulsabgabe kann gesteuert oder geregelt erfolgen. Für eine effektive biatriale Stimulation ist es zweckmäßig, die Reizschwelle für die linke und rechte Seite des Herzens getrennt zu bestimmen und/oder individuelle Pulsamplituden für das linke und rechte Atrium abzugeben. Aus diesem Grund ist die Steuer- oder Regeleinheit nunmehr derart ausgeführt, sodass die Stimulation des linken oder rechten Atriums wahlweise deaktivierbar ist. Damit kann gezielt entweder das linke oder rechte Atrium stimuliert werden. Für den Stimulationsbetrieb ist selbstverständlich eine parallele bzw. zeitgleiche oder verzögerte Stimulation beider Vorhöfe möglich.

Gemäß dem Anspruch 1 ist die Steuer- oder Regeleinheit derart ausgeführt, sodass im Stimulationsbetrieb für das linke und rechte Atrium eine gemeinsame Stimulationspulsamplitude einstellbar ist. Diese Möglichkeit kann bei getrennten Steuer- oder Regelkreisen bestehen, wenn für beide Steuer- oder Regelkreise ein einheitlicher Amplitudenwert einstellbar ist. Dabei kann die Steuer- oder Regeleinheit derart ausgeführt sein, sodass diese automatisch nach der erfolgten Reizschwellenbestimmung einen gemeinsamen Amplitudenwert für beide Steuer- oder Regelkreise konfiguriert.

Gemäß dem Anspruch 1 konfiguriert sich die Steuer- oder Regeleinheit automatisch in Abhängigkeit der Reizschwellenbestimmung, sodass die bestimmte und betragsmäßig größere Stimulationsamplitude der seitenindividuellen Reizschwellenbestimmung für die reguläre biatriale Stimulation verwendet wird. Folglich legt das Atrium, das die größere Amplitude für eine effektive Stimulation erfordert, die gemeinsame Stimulationsamplitude im Stimulationsbetrieb fest.

Der Herzschrittmacher gemäß der vorliegenden Erfindung kann als solcher ausgeführt sein oder mit einem Kardioversionsgerät bzw. Defibrillator ausgeführt bzw. verbunden sein.

Der Herzschrittmacher umfasst wenigstens zwei Stimulationsausgänge, die mit den entsprechenden Elektroden verbindbar sind. Die Stimulation kann als intrakardiale oder extrakardiale Stimulation erfolgen. Die Steuer- oder Regeleinheit kann hardwareseitig getrennte Steuer- bzw. Regelkreise für das linke und rechte Atrium aufweisen. Alternativ kann die Steuer- oder Regeleinheit hardwareseitig einen gemeinsamen Steuer- oder Regelkreis für das linke und rechte Atrium aufweisen, wobei die wenigstens zwei Stimulationsausgänge wahlweise deaktivierbar sind.

Die erfindungsgemäße Ausführung der Steuer- oder Regeleinheit eignet sich insbesondere für die individuelle Reizschwellenbestimmung von linkem und rechtem Atrium. Hierzu ist wahlweise die Stimulation des linken oder rechten Atriums deaktivierbar, um gezielt entweder das linke oder rechte Atrium stimulieren zu können. In der Regel wird die Impulsamplitude niedrig eingestellt und schrittweise erhöht bis die Effektivität der Stimulation mittels EKG-Monitoring erkennbar ist und so die Reizschwelle definiert.

Weiterhin kann es vorteilhaft sein, wenn die Steuer- oder Regeleinheit derart ausgeführt ist, sodass für den Stimulationsbetrieb unterschiedliche Pulsamplituden für das linke und rechte Atrium einstellbar sind. Dadurch lassen sich die Amplitudenwerte gezielt auf die seitenabhängige Reizschwelle definieren. Dies erfordert neben der Möglichkeit zur Deaktivierung der Stimulation bei biatrialen Stimulationsmodi darüber hinaus eine getrennte Steuerung oder Regelung der Pulsamplituden für beide Herzvorhöfe. Die getrennte Steuerung oder Regelung ist vorzugsweise hardwareseitig und/oder softwareseitig vorgesehen.

Die Steuer- oder Regeleinheit ist ausgeführt, im Stimulationsbetrieb für das linke und rechte Atrium eine gemeinsame Stimulationspulsamplitude einzustellen. Diese Möglichkeit kann bei getrennten Steuer- oder Regelkreisen bestehen, wenn für beide Steuer- oder Regelkreise ein einheitlicher Amplitudenwert einstellbar ist. Dabei ist die Steuer- oder Regeleinheit derart ausgeführt, dass diese automatisch nach der erfolgten Reizschwellenbestimmung einen gemeinsamen Amplitudenwert für beide Steuer oder Regelkreise konfiguriert.

Diese vorteilhafte Eigenschaft der Steuer- oder Regeleinheit ist jedoch auch dann zweckmäßig, wenn der Herzschrittmacher nicht die notwendigen Hardwarevoraussetzungen für die Abgabe von getrennten Stimulationspulsen für das rechte und linke Atrium bereitstellt. In diesem Fall ist es ausreichend, wenn die Steuer- oder Regeleinheit eine Deaktivierung des jeweiligen Stimulationspulses bzw. Stimulationsausgangs erlaubt. Die individuell bestimmten Reizschwellen für das linke und rechte Atrium definieren sodann einen gemeinsamen Wert für die Abgabe der Stimulationsamplitude.

Weiterhin kann die Steuer- oder Regeleinheit derart ausgeführt sein, sodass die eine oder mehreren bestimmten Stimulationspulsamplituden im Stimulationsbetrieb automatisch um einen definierbaren Sicherheitsbetrag erhöht werden. Hierdurch wird die Wahrscheinlichkeit für eine effektive Stimulation weiter erhöht, da ein gewisser Sicherheitsabstand zur jeweiligen Reizschwelle eingehalten wird.

In einer vorteilhaften Ausgestaltung der Erfindung umfasst der externe Herzschrittmacher ein Anzeigeelement zur Darstellung eines Konfigurationsmenüs. Das Konfigurationsmenü sieht eine Konfigurationsmöglichkeit für den Anwender vor, über die dieser wahlweise die linke oder rechte Atriumstimulation aktivieren bzw. deaktivieren kann.

Zur Sicherheit ist das Konfigurationsmenü sinnvollerweise derart ausgeführt, sodass immer nur eine Deaktivierung des linken oder rechten Atriums zugelassen ist. Dies bedeutet, dass entweder wenigstens eine Pulsabgabe für das linke oder rechte Atrium aktiv sein muss. Eine Deaktivierung beider Pulsabgaben ist unzulässig und wird dem Benutzer aktiv verwehrt.

Der externe Herzschrittmacher bzw. die Steuer- oder Regeleinheit kann weiterhin Mittel aufweisen, die eine getriggerte und / oder gehemmte Pulsabgabe ausführen. Während des inhibierten Modus wird die Abgabe eines Impulses bei herzeigener Aktivität unterdrückt, während im getriggerten Modus ein im Atrium registriertes Signal zur Pulsabgabe führt. Offensichtlich muss die Steuer- oder Regeleinheit daher Mittel umfassen, die eine Detektion der Herzaktivität erlaubt. In an sich bekannter Art und Weise kann die Wahrnehmungsschwelle für die Detektierung der herzeigenen Aktivität ermittelbar und einstellbar sein.

In einer weiteren vorteilhaften Ausgestaltung der Erfindung kann die Steuer- oder Regeleinheit Mittel für die zeitverzögerte Pulsabgabe aufweisen. Hierdurch besteht die Möglichkeit, dass die Stimulationspulse für das linke und rechte Atrium zeitverzögert zueinander abgebbar sind. Die Zeitverzögerung kann weiterhin konfigurierbar sein.

Da die Steuer- oder Regeleinheit möglicherweise die Stimulationspulsamplitude automatisch in Abhängigkeit der beiden individuell bestimmten Reizschwellen festlegt, ist es zweckmäßig, wenn dem Anwender über das Anzeigeelement wenigstens eine Hinweisnachricht eingeblendet wird, die den Anwender über die Verwendung einer gemeinsamen Impulsamplitude für beide Vorhöfe und/oder die Höhe der verwendeten Amplitude in Kenntnis setzt. Diese Benachrichtigung ist insbesondere dann sinnvoll, wenn bei der Reizschwellenbestimmung durch den Anwender unterschiedliche Pulsamplituden festgelegt worden sind. Die Steuer- oder Regeleinheit überschreibt diese anwenderseitig konfigurierten Werte mit dem automatisch bestimmten Wert der gemeinsamen Stimulationspulsamplitude.

Neben dem externen Herzschrittmacher ist ein Verfahren zur Reizschwellenbestimmung mit Hilfe des externen Herzschrittmachers bzw. einer vorteilhaften Ausgestaltung des Herzschrittmachers vorgesehen. Gemäß dem Verfahren wird eine atriale Stimulationspulsabgabe abwechselnd für das linke und rechte Atrium deaktiviert und die Reizschwelle individuell für das linke und rechte Atrium bestimmt. Bei dieser Vorgehensweise wird zuerst die Impulsabgabe für das linke oder rechte Atrium deaktiviert und eine niedrige Pulsamplitude für das aktivierte Atrium eingestellt. Diese wird schrittweise so lange erhöht, bis eine effektive Stimulation erkannt werden kann. Insbesondere wird eine effektive Stimulation mittels eines EKG-Monitors bzw. EKG-Schreibers überprüft und erkannt. Die gewählte Stimulationsrate bei der Reizschwelleneinstellung muss notwendigerweise über der Eigenfrequenz des Herzens liegen.

Sofern die Reizschwelle für das erste Atrium erkannt worden ist, wird der andere Vorhof aktiviert und in selbiger Weise die Reizschwelle bestimmt.

Da die Reizschwellen des linken und rechten Atriums üblicherweise voneinander abweichen, wird gemäß einer vorteilhaften Ausführung des Verfahrens eine gemeinsame Stimulationspulsamplitude für die biatriale Stimulation, d.h. für die Stimulation des linken und rechten Atriums verwendet. Insbesondere wird die gemeinsame Pulsamplitude gewählt, die der betragsmäßig höheren Reizschwelle der individuell bestimmten Reizschwellen entspricht.

Weiterhin kann es zweckmäßig sein, dass die gemeinsame Pulsamplitude für den Stimulationsbetrieb um einen gewissen Pufferbetrag erhöht wird, um einen ausreichenden Sicherheitsabstand zur jeweiligen Reizschwelle des linken und rechten Atriums zu haben.

Die Erfindung betrifft des Weiteren eine auf einem Datenträger gespeicherte Steuerungssoftware zur Durchführung des Verfahrens auf einem externen Herzschrittmacher gemäß der vorliegenden Erfindung. Die Steuerungssoftware weist offensichtlich dieselben Vorteile und Eigenschaften wie das Verfahren bzw. der externe Herzschrittmacher auf, weshalb an dieser Stelle auf eine wiederholende Beschreibung verzichtet werden soll.

Weitere Vorteile und Eigenschaften der Erfindung sollen im Folgenden anhand eines in den Figuren dargestellten Ausführungsbeispiels näher erläutert werden. Es zeigen:

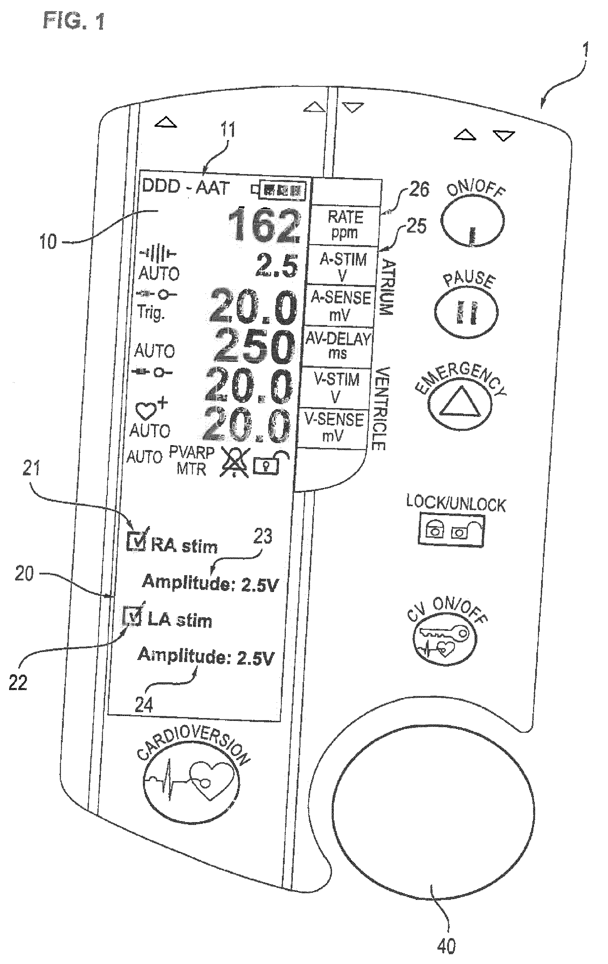

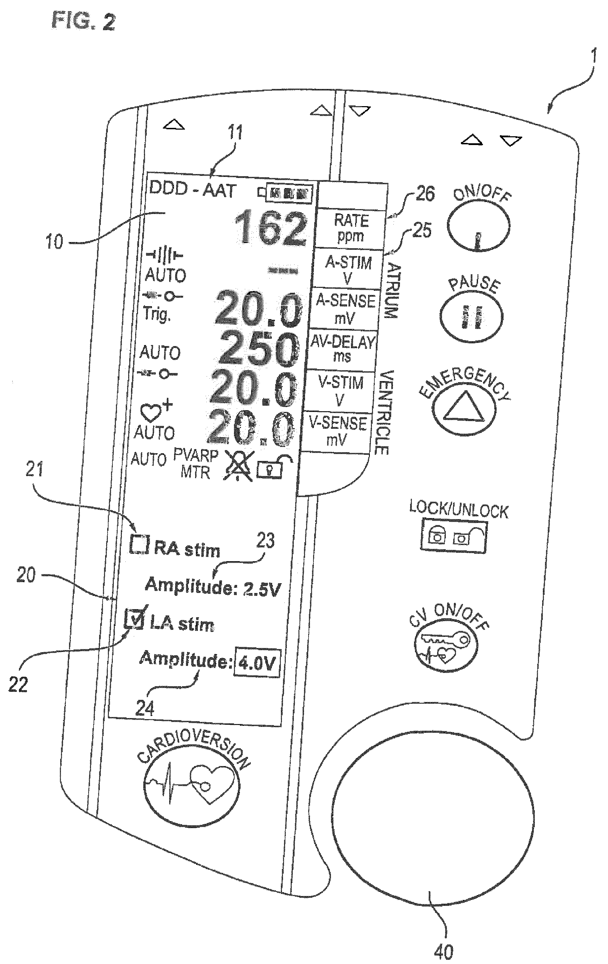

- Fig. 1,2:: skizzenhafte Darstellungen des erfindungsgemäßen externen Herzschrittmachers.

Den Figuren 1 und 2 ist eine skizzenhafte Draufsicht auf den erfindungsgemäßen externen Herzschrittmacher 1 zu entnehmen. Das Gerät 1 weist ein Anzeigeelement 10 auf, das sich links auf der Geräteoberseite befindet. Das Anzeigeelement 10 dient unter Anderem zur Darstellung des Konfigurationsmenüs, um individuelle Einstellungen bezüglich des auszuführenden Herzschrittmachermodus und patientenindividuelle Anpassung für den gewählten Modus vornehmen zu können.

Beide Figuren zeigen den Menübereich zur vorhofseparaten Reizschwellenbestimmung für biatriale Stimulationsmodi. Der dargestellte Menübereich ist nur dann zugänglich, wenn vorab der passende Stimulationsmodus ausgewählt wurde, d.h. ein biatrialer Stimulationsmodus selektiert wurde. Der gewählt Modus 11 wird in der obersten Displayzeile am linken Displayrand eingeblendet.

Im unteren Displaybereich 20 stehen die beiden Checkboxen 21 und 22 zur Aktivierung/Deaktivierung zur Verfügung. Die obere Checkbox 21 aktiviert bei gesetztem Haken die Stimulation des rechten Atriums, während die untere Checkbox bei aktiviertem Haken die Stimulation des linken Atriums bewirkt. Eine Deaktivierung wird durch Entfernen des Hakens erzielt. Die Auswahl und Aktivierung/Deaktivierung erfolgt über den Drehknopf 40, der sowohl auf Drehbetätigung als auch auf Druckkraft reagiert.

Beim Betreten des dargestellten Menüs sind beide Checkboxen 21, 22 standardmäßig gesetzt, d.h. die Pulsabgabe ist sowohl für das linke als auch rechte Atrium aktiviert.

Über die Felder 23, 24 unterhalb der Checkboxen 21, 22 wird die gewählte Stimulationsamplitude für das linke und rechte Atrium angezeigt als auch konfiguriert. Beim erstmaligen Betreten des Konfigurationsmenüs wird der initiale Wert aus dem Feld "A STIM V" 25 übernommen, das die aktive Stimulationspulsamplitude im Stimulationsbetrieb des Herzschrittmachers mit biatrialer Stimulation kennzeichnet. Im Beispiel der Figur 1 beträgt der Wert 2.5 V, der automatisch in die Felder 23, 24 für die Amplituden zur Reizschwellenbestimmung übernommen wird.

Für eine effektive biatriale Stimulation mit Hilfe des externen Herzschrittmachers 1 muss für jedes Atrium eine individuelle Reizschwelle bestimmt werden. Nach Auswahl eines biatrialen Stimulationsmodus, angezeigt im Feld 11, deaktiviert der Anwender durch Entfernen des Hakens der Checkbox 21 die Stimulation des rechten Herzvorhofs. Dabei wählt der Anwender über den Drehknopf 40 die Checkbox 21 an und entfernt durch Druckbetätigung den Haken. Nochmalige Druckbetätigung setzt den Haken erneut. Die entsprechende Menüdarstellung ist der Figur 2 zu entnehmen.

Das Menü symbolisiert dem Anwender, dass während der Ausführung der Reizschwellenbestimmung nur noch ein Puls am linken Atrium abgegeben wird. Der Anwender wählt zunächst eine niedrige Pulsamplitude für die Stimulierung des linken Atriums. Die Auswahl der Stimulationsamplitude 24 erfolgt durch die Druckbetätigung des Drehknopfes 40, wodurch das ausgewählte Feld 24 invertiert dargestellt wird. Über die Drehbewegung des Drehknopfes 40 kann die Amplitude erhöht bzw. erniedrigt werden. Im Feld "A STIM" 25 wird während der Durchführung der Reizschwellenbestimmung der Wert "---" angezeigt.

Die Amplitude für die Stimulation des linken Atriums wird nun kontinuierlich bzw. stufenartig erhöht und gleichzeitig das Patientenherz über einen externen EKG-Monitor bzw. einen EKG-Schreiber auf eine effektive Stimulation beim Auslösen eines Stimulationsimpulses hin überwacht. Der kleinste Amplitudenwert bei dem eine effektive Stimulation erkannt wird, definiert die Reizschwelle. Als notwendige Voraussetzung für die Bestimmung der Reizschwelle muss eine Stimulationsrate im Feld 26 ausgewählt werden, die überhalb der Eigenfrequenz des Herzens liegt.

Sobald die entsprechende Amplitude für die Reizschwelle des linken Atriums gefunden wurde, in der Figur 2 liegt diese bei 4V, wird die Stimulation für das rechte Atrium über die Checkbox 21 aktiviert und die Checkbox 22 für das linke Atrium deaktiviert. Aus Sicherheitsgründen ist ein Deaktivieren beider Kanäle 21, 22 nicht möglich, weshalb immer erst beide Checkboxen 21, 22 aktiviert seien müssen, bevor eine der beiden Checkboxen 21, 22 wieder deaktiviert werden kann.

Nach Wiederholung des beschriebenen Vorgehens für das rechte Atrium ist die Bestimmung der Reizschwellen für beide Vorhöfe abgeschlossen. Da die beiden ermittelten Reizschwellen meist unterschiedlich hoch sind, wird beim Verlassen des Einstellmenüs automatisch eine gemeinsame Pulsamplitude für beide Vorhöfe gewählt, die mit der höheren der beiden ermittelten Reizschwellen identisch ist. Dieser Wert wird für den Stimulationsbetrieb in das Feld 25 automatisch übernommen.

Optional kann eine automatische Erhöhung der Pulsamplitude um einen relativen oder absoluten Wert erfolgen, um durch einen Sicherheitspuffer einer nachträglichen, physiologisch bedingten Erhöhung der Reizschwelle Rechnung zu tragen. Die tatsächlich eingestellte Pulsamplitude wird dem Anwender durch eine Meldung beim Verlassen des Menüs eingeblendet, die er vor Aufnahme der regulären biatrialen Stimulation bestätigen muss. Diese eingestellte Amplitude wird nach Verlassen des Menüs dauerhaft an einer vorgegebenen Position im Display des externen Herzschrittmachers angezeigt.

Der erfindungsgemäße Gedanke des externen Herzschrittmachers liegt in der Möglichkeit zum Deaktivieren der Stimulation des linken oder rechten Atriums während eines biatrialen Stimulationsmodus. Die Deaktivierung kann sowohl in Hardware, als auch in Software erfolgen. Aufgrund der Verwendung einer gemeinsamen Pulsamplitude für die Stimulation der beiden Vorhöfe sind auch externe Herzschrittmacherbauarten für die Erfindungsumsetzung geeignet, die aufgrund ihrer Hardwarebeschaffenheit im Stimulationsbetrieb nur eine gemeinsame Pulsamplitude einstellen können und bei denen der externe Herzschrittmacher die stimulierte Herzaktivität aufgrund der Eigenschaften der Hardwareschaltung nur in einem oder in keinem der beiden Vorhöfe wahrnehmen kann.

## Patentansprüche

1. Externer Herzschrittmacher zur biatrialen Stimulierung, mit einer Steuer- oder Regeleinheit zur Abgabe wenigstens eines Stimulationspulses, wobei die Stimulation des linken oder rechten Atriums wahlweise deaktivierbar ist, **dadurch gekennzeichnet, dass** die Steuer- oder Regeleinheit ausgeführt ist, für den Stimulationsbetrieb für das linke und rechte Atrium eine gemeinsame Stimulationspulsamplitude einzustellen, und dass die Steuer- oder Regeleinheit ausgeführt ist, automatisch die aus der Reizschwellenbestimmung bestimmte und betragsmäßig größere Stimulationspulsamplitude für die biatriale Stimulation des linken und rechten Atriums zu verwenden, wobei das Atrium, das die größere Amplitude für eine effektive Stimulation erfordert, die gemeinsame Stimulationsamplitude im Stimulationsbetrieb festlegt.

2. Externer Herzschrittmacher nach Anspruch 1, **dadurch gekennzeichnet, dass** die Stimulation des linken oder rechten Atriums für die Reizschwellenbestimmung wahlweise deaktivierbar ist, wobei die Impulsamplitude niedrig einstellbar und schrittweise erhöhbar ist, bis die Effektivität der Stimulation mittels EKG-Monitoring erkennbar und so die Reizschwelle definierbar ist.

3. Externer Herzschrittmacher nach einem der vorhergehenden Ansprüche, **dadurch gekennzeichnet, dass** die Steuer- oder Regeleinheit derart ausgeführt ist, so dass die ein oder mehreren bestimmten Stimulationspulsamplituden im Stimulationsbetrieb automatisch um einen definierbaren Sicherheitsbetrag erhöht werden.

4. Externer Herzschrittmacher nach einem der vorhergehenden Ansprüche, **dadurch gekennzeichnet, dass** dieser ein Anzeigeelement zur Darstellung eines Konfigurationsmenüs aufweist, wobei das Konfigurationsmenü eine Konfigurationsmöglichkeit für den Anwender zur Aktivierung und Deaktivierung der linken und rechten Atriumstimulation umfasst.

5. Externer Herzschrittmacher nach Anspruch 4, **dadurch gekennzeichnet, dass** das Konfigurationsmenü nur eine Deaktivierung entweder des linken oder des rechten Atriums zulässt.

6. Externer Herzschrittmacher nach einem der vorhergehenden Ansprüche, **dadurch gekennzeichnet, dass** die Steuer- oder Regeleinheit Mittel für die getriggerte und/oder gehemmte Impulsabgabe aufweist.

7. Externer Herzschrittmacher nach einem der vorhergehenden Ansprüche, **dadurch gekennzeichnet, dass** die Steuer- oder Regeleinheit Mittel für die zeitverzögerte Stimulationspulsabgabe aufweist, wodurch die Stimulationspulse des linken und rechten Atriums zeitverzögert zueinander abgebbar sind.

8. Externer Herzschrittmacher wenigstens nach den Ansprüchen 1 und 4. **dadurch gekennzeichnet, dass** die Steuer- oder Regeleinheit ausgeführt ist, dem Anwender die automatisch bestimmte gemeinsame Stimulationspulsamplitude für die biatriale Stimulation per Benachrichtigung, insbesondere per optischer und/oder akustischer Benachrichtigung, am Anzeigeelement mitzuteilen.

9. Auf einem Datenträger gespeicherte Steuerungssoftware zur Durchführung eines Verfahrens zur Reizschwellenbestimmung auf einem externen Herzschrittmacher nach einem der Ansprüche 1 bis 8, wobei das Verfahren die folgenden Schritte aufweist:
Abwechselnde Deaktivierung der atrialen Simulationspulsabgabe für das linke und das rechte Atrium;
Individuelle Bestimmung einer Reizschwelle für das linke und rechte Atrium;
Einstellen einer gemeinsamen Stimulationspulsamplitude für das linke und rechte Atrium für den Stimulationsbetrieb durch eine Steuer- oder Regeleinheit des externen Herzschrittmachers;
wobei die aus der Reizschwellenbestimmung bestimmte und betragsmäßig größere Stimulationspulsamplitude für die biatriale Stimulation des linken und rechten Atriums automatisch durch die Steuer- oder Regeleinheit verwendet wird; wobei das Atrium, das die größere Amplitude für eine effektive Stimulation erfordert, die gemeinsame Stimulationsamplitude im Stimulationsbetrieb festlegt.

## Claims

1. An external pacemaker for biatrial stimulation, having a control or regulation unit for emitting at least one stimulation pulse, wherein the stimulation of the left or right atrium can selectively be deactivated, **characterized in that** the control or regulation unit is configured to set a common stimulation pulse amplitude for the left and right atria for the stimulation operation; and **in that**
the control or regulation unit is configured to automatically use the stimulation pulse amplitude determined from the stimulation threshold determination which is larger in amount for the biatrial stimulation of the left and right atria,
with the atrium which requires the larger amplitude for an effective stimulation consequently fixing the common stimulation amplitude in stimulation operation.

2. An external pacemaker in accordance with claim 1, **characterized in that** the stimulation of the left or right atria can be selectively deactivated for the determination of the stimulation threshold, with the pulse amplitude being able to be set as low and being able to be increased in steps until the effectiveness of the stimulation can be recognized by means of ECG monitoring and the stimulation threshold can thus be defined.

3. An external pacemaker in accordance with one of the preceding claims, **characterized in that** the control or regulation unit is configured such that the one or more determined stimulation pulse amplitudes are automatically increased by a definable safety amount in stimulation operation.

4. An external pacemaker in accordance with one of the preceding claims, **characterized in that** it has a display element for presenting a configuration menu, with the configuration menu comprising a configuration option for the user for activating and deactivating the left and right atrium stimulation.

5. An external pacemaker in accordance with claim 4, **characterized in that** the configuration menu allows a deactivation of only either the left or of the right atrium.

6. An external pacemaker in accordance with one of the preceding claims, **characterized in that** the control or regulation unit has means for the triggered and/or inhibited pulse emission.

7. An external pacemaker in accordance with one of the preceding claims, **characterized in that** the control or regulation unit has means for the time-delayed stimulation pule emission, whereby the stimulation pulses of the left and right atria can be emitted with a time delay with respect to one another.

8. An external pacemaker at least in accordance with claims 1 and 4, **characterized in that** the control or regulation unit is configured to inform the user of the automatically determined common pulse amplitude for biatrial stimulation by a message, in particular by an optical and/or acoustic message, at the display element.

9. Control software stored on a data carrier for carrying out a method for determining a stimulation threshold on an external pacemaker in accordance with one of the claims 1 to 8,
wherein the method comprises the following steps:
alternating deactivation of the atrial stimulation pulse emission for the left and right atria;
individual determination of a stimulation threshold for the left and right atria;
setting a common stimulation pulse amplitude for the left and right atria for the stimulation operation by a control or regulation unit of the external pacemaker;
wherein the stimulation pulse amplitude determined from the stimulation threshold determination and larger in amount is automatically used for the biatrial stimulation of the left and right atria by the control or regulation unit; and wherein the atrium which requires the larger amplitude for an effective stimulation fixes the common stimulation amplitude in stimulation operation.

## Revendications

1. Stimulateur cardiaque externe pour stimulation biauriculaire, comprenant une unité de commande ou de régulation destinée à émettre au moins une impulsion de stimulation, la stimulation de l'oreillette gauche ou l'oreillette droite pouvant être désactivée au choix, **caractérisé en ce que** l'unité de commande ou de régulation est exécutée pour régler une amplitude d'impulsion de stimulation commune pour l'oreillette gauche et l'oreillette droite pour le fonctionnement de stimulation, et **en ce que**
l'unité de commande ou de régulation est exécutée pour utiliser automatiquement l'amplitude d'impulsion de stimulation déterminée à partir de la détermination du seuil d'excitation et dont la valeur est la plus élevée pour la stimulation biauriculaire de l'oreillette gauche et l'oreillette droite,
dans lequel l'oreillette qui nécessite la plus grande amplitude pour une stimulation efficace définit l'amplitude de stimulation commune en fonctionnement de stimulation.

2. Stimulateur cardiaque externe selon la revendication 1, **caractérisé en ce que** la stimulation de l'oreillette gauche ou l'oreillette droite peut être désactivée au choix pour la détermination du seuil d'excitation, dans lequel
l'amplitude d'impulsion peut être réglée à un niveau bas et augmentée petit à petit, jusqu'à ce que l'efficacité de la stimulation puisse être détectable au moyen du contrôle de l'E.C.G. et que le seuil d'excitation soit ainsi définissable.

3. Stimulateur cardiaque externe selon l'une des revendications précédentes, **caractérisé en ce que** l'unité de commande ou de régulation est exécutée de telle manière que la ou les plusieurs amplitudes d'impulsion de stimulation déterminées sont augmentées automatiquement d'une valeur de sécurité définissable en fonctionnement de stimulation.

4. Stimulateur cardiaque externe selon l'une des revendications précédentes, **caractérisé en ce que** celui-ci comporte un élément d'affichage destiné à représenter un menu de configuration, le menu de configuration comprenant une option de configuration pour l'utilisateur afin d'activer et désactiver la stimulation de l'oreillette gauche et l'oreillette droite.

5. Stimulateur cardiaque externe selon la revendication 4, **caractérisé en ce que** le menu de configuration autorise uniquement une désactivation soit de l'oreillette gauche, soit de l'oreillette droite.

6. Stimulateur cardiaque externe selon l'une des revendications précédentes, **caractérisé en ce que** l'unité de commande ou de régulation comporte des moyens pour l'émission d'impulsions déclenchée et/ou inhibée.

7. Stimulateur cardiaque externe selon l'une des revendications précédentes, **caractérisé en ce que** l'unité de commande ou de régulation comporte des moyens pour l'émission temporisée d'impulsions de stimulation, moyennant quoi les impulsions de stimulation de l'oreillette gauche et l'oreillette droite peuvent être émises de manière décalée dans le temps l'une par rapport à l'autre.

8. Stimulateur cardiaque externe au moins selon les revendications 1 et 4, **caractérisé en ce que** l'unité de commande ou de régulation est exécutée pour informer l'utilisateur de l'amplitude d'impulsion de stimulation commune déterminée automatiquement pour la stimulation biauriculaire par notification, en particulier par notification optique et/ou acoustique sur l'élément d'affichage.

9. Logiciel de commande enregistré sur un support de données, destiné à exécuter un procédé de détermination du seuil d'excitation sur un stimulateur cardiaque externe selon l'une des revendications 1 à 8, le procédé comportant les étapes suivantes consistant à :
désactiver alternativement l'émission d'impulsions de stimulation auriculaire pour l'oreillette gauche et l'oreillette droite ;
déterminer individuellement un seuil d'excitation pour l'oreillette gauche et l'oreillette droite ;
régler une amplitude d'impulsion de stimulation commune pour l'oreillette gauche et l'oreillette droite pour le fonctionnement de stimulation au moyen d'une unité de commande ou de régulation du stimulateur cardiaque externe ;
dans lequel l'amplitude d'impulsion de stimulation déterminée à partir de la détermination du seuil d'excitation et dont la valeur est la plus élevée est utilisée automatiquement par l'unité de commande ou de régulation pour la stimulation biauriculaire de l'oreillette gauche et l'oreillette droite ; l'oreillette qui nécessite la plus grande amplitude pour une stimulation efficace définissant l'amplitude d'impulsion de stimulation commune en fonctionnement de stimulation.
